# EUROPÄISCHE PATENTSCHRIFT

(11) **EP 0 469 273 B1**
(45) Veröffentlichungstag und Bekanntmachung des Hinweises auf die Patenterteilung: **10.12.2003**
(21) Anmeldenummer: 91109934.9
(22) Anmeldetag: 18.06.1991
(51) Int. Cl.: C12N 15/82, C12N 5/10, C12N 15/54, A01H 5/00

(54) **Fertile transgene Maispflanzen mit artfremdem Gen sowie Verfahren zu ihrer Herstellung**
Fertile transgenic maize plants with foreign gene as well as method for their production
Mais transgéniques fertiles contenant un gène étranger et méthode pour leur production

(30) Priorität: 23.06.1990 EP 90111946
(43) Veröffentlichungstag der Anmeldung: 05.02.1992
(73) Patentinhaber: Bayer CropScience GmbH, 65929 Frankfurt/Main (DE)
(72) Erfinder: Dudits, Denes, Dr., H-6726 Szeged (HU); Morocz, Sandor, Dr., H-6726 Szeged (HU); Nemeth, Janos, Dr., H-6726 Szeged (HU); Donn, Günther, Dr., W-6238 Hofheim am Taunus (DE)

(56) Entgegenhaltungen:
- EP-A- 0 290 987
- EP-A- 0 292 435
- EP-A- 0 348 348
- ABSTRACTS VIITH IAPTC CONGRESS June 24-29 1990, Seite 53; DONN, G. et al.: "Stable transformation of maize with a chimaeric, modified Phosphinothricin-acetyltransferase gene from Streptomyces viridochromogenes".
- SCIENCE, Bd. 240, 8. April 1988, Seiten 204-207; RHODES, C.A. et al.: "Genetically transformed maize plants from protoplasts"
- IN VITRO CELL DEV BIOL, Bd. 26, Maerz 1990, (3 Part 2) Tagung Juni 10-13, 1990, Seite 33A; GORDON-KAMM, W.J. et al.: "Transformation of corn using microprojectile bombardment"

## Beschreibung

Dikotyle Pflanzen sind im wesentlichen über Ti-Plasmid-Vektorsysteme mit Hilfe von Agrobacterium tumefaciens transformierbar. Dieses System ist jedoch bei monokotylen Pflanzen nicht ohne weiteres anzuwenden. Potrykus et al. (Mol. Gen. Genet. 199, 183 (1985)) und Lörz et al. (Mol. Gen. Genet. 199, 178, (1985)) zeigten, daß Pflanzenprotoplasten monokotyler Pflanzen fremde DNA stabil in ihr Genom integrieren können. Fortschrittsinhibierend war jedoch zunächst die fehlende Regenerierbarkeit der Protoplasten zu fertilen Pflanzen.

In den letzten Jahren wurden daher intensive Anstrengungen unternommen, Genotypen und Verfahren ausfindig zu machen, die es ermöglichen, das Problem der Pflanzenregeneration in den Griff zu bekommen. In EP 292 435 wird ein Verfahren beschrieben mit Hilfe dessen, ausgehend von einem schleimlosen, weichen (friable) granulösen Mais-Kallus, fertile Pflanzen erhalten werden können. Shillito et al. (Bio/Technology 7, 581 (1989)) haben in diesem Zusammenhang beobachtet, daß es ferner für die Regenierbarkeit zu fertilen Pflanzen notwendig ist, von Kallus-Suspensionskulturen auszugehen, aus denen eine sich teilende Protoplastenkultur, mit der Fähigkeit zu Pflanzen zu regenerieren, herstellbar ist.

Nach einer in vitro Kultivierungszeit von 7 bis 8 Monaten erhalten Shillito et al. Pflanzen mit lebensfähigen Nachkommen, die jedoch Abnormalitäten in der Morphologie und der Reproduktivität aufweisen.

Prioli und Söndahl (Bio/Technology 7, 589 (1989)) beschreiben die Regeneration und die Gewinnung fertiler Pflanzen aus Mais-Protoplasten der Cateto Mais-Inzuchtlinie Cat 100-1. Die Autoren vermuten, daß die Protoplasten-Regeneration zu fertilen Pflanzen abhängig ist von einer Anzahl verschiedener Faktoren, wie z.B. vom Genotyp, vom physiologischen Zustand der Donor-Zellen und von den Kultivierungsbedingungen.

In der Europäischen Patentanmeldung 90 111 945.3 [Titel: Improved Zea mays (L.) genotypes with capability of Long term, Highly Efficient Plant Regeneration; wurde zeitgleich mit vorliegender Anmeldung eingereicht] zur HOE 90/F 184 wird ein neuer Maisgenotyp beschrieben, von dem ausgehend reproduzierbar in kurzer Zeit Protoplasten hergestellt werden können, die zu fertilen Pflanzen ohne Abnormalitäten regenerieren können.

Der neue Maisgenotyp unterscheidet sich von bisher beschriebenen regenerierbaren Genotypen dadurch, daß sich ausgehend von unreifen Embryonen auf hormonfreien Nährmedien an den sich entwickelnden Keimpflanzen an der Sproßbasis ein Auxin autotropher embryogener Kallus bildet. Dieser Kallus läßt sich über einen langen Zeitraum (> 12 Monate) auf hormonfreien Medien subkultivieren unter Beibehaltung seiner Fähigkeit zur Embryogenese. Unter diesen Kulturbedingungen bilden sich voll entwickelte Embryonen, die gelegentlich spontan zu Pflanzen ausdifferenzieren. Außerdem entstehen Adventivembryonen in großer Zahl, besonders bei erhöhtem Saccharosegehalt (6-9 %) im Medium. Durch den Wechsel des Nährmediums (Reduktion des Saccharosegehalts auf 2-3 % und Zugabe von 1-3 mg/l 2,4 Dichlorphenoxyessigsäure (2,4-D) bzw. Dicamba) läßt sich reproduzierbar die Bildung von weichem, granulösem Kallus, der aus embryogenen Zellaggregaten besteht (Typ II-Kallus) induzieren. Dieser Typ II-Kallus kann in Flüssigmedien als Zellsuspensionskultur subkultiviert werden und eignet sich zur Isolierung von totipotenten Protoplasten. Die Protoplasten können u.a. für die genetische Transformation verwendet werden und können unter den in der Erfindung beschriebenen Bedingungen zu fertilen transgenen Maispflanzen regeneriert werden.

Die Erfindung betrifft somit:
1. Eine transgene Maiszellinie, hergestellt aus Protoplasten, die aus einem auxinautotrophen Maisgenotyp gewonnen wurden, sowie aus dieser Maiszellinie regenerierte transgene Pflanzen und Pflanzenteile und deren Nachkommen.
2. Ein Verfahren zur Herstellung der transgenen Maiszellinie, das dadurch gekennzeichnet ist, daß
   - aus Zellsuspensionen des Maisgenotyps enzymatisch Protoplasten hergestellt werden
   - die Protoplasten mit DNA inkubiert werden
   - die transgene Maiszellinie aus den DNA enthaltenden Protoplasten selektiert und regeneriert wird.

Wie in der Europäischen Patentanmeldung 90 111 945.3 erwähnt, wurde die in der bevorzugten Ausführungsform eingesetzte hoch embryogene Mais-Zellinie DSM 6009 bei der Deutschen Sammlung von Mikroorganismen und Zellkulturen GmbH am 1.6.1990 unter den Bedingungen des Budapester Vertrags mit dem genannten Aktenzeichen hinterlegt. Wie in der genannten Patentanmeldung beschrieben, wurde die Zellinie aus dem Embryo einer Maispflanze gewonnen, die durch Kreuzung der Genotypen H 229 und OK 281 erhalten wurde. Zur Herstellung der erfindungsgemäßen transgenen Maispf lanzen werden Protoplasten aus Suspensionskulturen der Zellinie hergestellt, die dann mit der zu inkorporierenden DNA inkubiert werden.

Die Herstellung der Protoplasten aus DSM 6009 erfolgt mit Hilfe von lysierenden Enzymen, wie z.B. Cellulasen und Pectinasen, wobei die Enzyme auch als Mischungen verwendet werden können. Die Konzentration der Enzyme kann in weiten Grenzen von 0,05-4 % (Gewicht pro Volumen Enzymlösung) schwanken. Die optimale Zusammensetzung der wäßrigen osmotischen Lösung sowie die Enzymkonzentration muß für den jeweiligen Gewebetyp in einfachen Versuchen ermittelt werden.

Zur Stabilisierung der Protoplasten muß die wäßrige Lösung osmotisch aktive Substanzen wie Mannit, Sorbit, Glucose, Saccharose, Fructose, KNO₃ oder andere typische Salze von Pflanzennährmedien enthalten. Die Osmolarität der Enzymlösung liegt vorteilhaft zwischen 500 und 750 mOsm, vorzugsweise zwischen 600 und 700 mOsm. Der pH-Wert kann zwischen pH 4,5 und 6,0 variieren. Vorzugsweise wird die Enzymlösung auf pH 5,5 bis 6 eingestellt und mit 1 bis 5 mM Phosphat und 1 bis 10 mM Morpholinoethansulfonsäure (MES) stabilisiert.

In Abhängigkeit von der Konzentration der lysierenden Enzyme werden die Protoplasten innerhalb von 3-20 h freigesetzt. Vorsichtiges Schütteln während der Enzyminkubation ist vorteilhaft. Das Schütteln auf einem Rundschüttler bei 10-50 rpm hat sich bewährt.

Es kann jegliche DNA in die Zelle eingeschleust werden. Vorteilhaft wird mit selektierbaren Markergenen gearbeitet, wie z.B. das Gen für Phosphinothricinacetyltransferase, Acetyl-coA-Carboxylase, Glutathion-S-transferase, Hygromycinphosphotransferase, Acetolactatsynthetase, 5-Enolpyruvyl-shikimat-phosphatsynthetase (EPSP-Synthetase), Glutaminsynthetase oder Transaminase. Es können natürlich auch Gene eingeschleust werden, deren Expression nicht durch Wachstum der Pflanze selektiert werden kann. Derartige Gene können beispielsweise für das δ-Endotoxin von Bacillus thuringiensis kodieren oder für Virusresistenz, wie z.B. Gene für Virus "coat" Proteine. Ferner kann das Chitinase Gen oder Gene, die die photosynthetische Leistungsfähigkeit der Pflanze verbessern, wie z.B. das Gen für Phosphoenolpyruvatcarboxylase oder bakterielle Asparaginsynthetase (Nakamura, M.M. et al.

Nucleic Acids Research 9, 4669 (1981)) eingesetzt werden. Bevorzugt wird mit dem Gen für die Phosphinothricinacetyltransferase aus Streptomyces viridochromogenes (Wohlleben, W. et. al., Gene 80, 25-57 (1988) gearbeitet, das für eine Expression in Pflanzen entsprechend modifiziert werden kann. Durch den Einbau dieses Gens werden die Maispflanzen und ihre Nachkommen resistent gegen Phosphinothricin (Glufosinate) und Phosphinothricinalanylalanin (Bialaphos).

Nach Synthetisierung bzw. Isolierung kann das Gen nach herkömmlichen Methoden, z.B. durch Transformation von E. coli oder durch "polymerase chain reaction", vermehrt werden. Das isolierte funktionelle Gen kann dann direkt oder auf einem beliebigen Klonierungsvektor, vorteilhaft pBR 322, pUC 18 oder pDH 51 (Pietrzak, M. et al. Nucl. Acid Res. 5858 (1986)) mit oder ohne einen in Pflanzen wirksamen Promotor in die Protoplasten eingeschleust werden. Als Promotor wird bevorzugt der 35S Promotor verwendet. Die DNA kann auch in Form eines Komplexes mit Histonen eingesetzt werden, wie es z.B. in der Deutschen Patentanmeldung 40 05 152.8 beschrieben wird.

Zur Protoplasteninkubation wird die DNA in wäßriger Lösung, die auch Puffersalze enthalten kann, suspendiert und zu den Protoplasten, die sich in einem osmot isch wirkenden Transformationspuffer befinden, gegeben.

Der Transformationspuffer besteht aus einer wäßrigen Lösung von Salzen, vorzugsweise zweiwertiger Kationen wie MgCl₂ oder CaCl₂ bzw. aus deren Gemischen oder auch von Zuckern, wie Saccharose, Glucose oder Fructose, oder Zuckeralkoholen, z.B. Mannit oder Sorbit. Die Salze befinden sich in einer Menge von 30-300 mval, die Zucker bzw. die Zuckeralkohole in einer Konzentration von 50 bis 500 mmol in dem Transformationspuffer.

Nach Zugabe der DNA-Lösung zu den Protoplasten wird Polyethylenglykol, vorteilhaft PEG 1500 bis 8000, zu der Mischung gegeben, so daß die Endkonzentration ca. 10 bis 25 Gewichts-%, bevorzugt 6 bis 10 Gewichts-% pro 1 Mischung beträgt. Es kann dann bei einem pH-Wert von 6,0 bis 8,0, vorzugsweise 6,5 bis 7, und einer Temperatur von 15 bis 40°C, vorzugsweise 20 bis 25°C, über einen Zeitraum von 5 Minuten bis zu einer Stunde, bevorzugt 20 bis 30 Minuten inkubiert werden. Nach beendeter Inkubation werden die Zellen vorteilhaft auf ein Wachstumsmedium überimpft, das anorganische Salze, z.B. Ammonium-, Kalium-, Calciumund Magnesiumsalze sowie Nitrat, Phosphat, Sulfat, Dihydrogenphosphat, Citrat, Vitamine, Aminosäuren, ein synthetisches Auxin, z.B. 2,4-D oder Dicamba sowie Zucker, vorzugsweise eine Mischung aus Fructose, Glucose und Saccharose, enthält, nachdem zuvor das PEG durch einen oder mehrere Waschvorgänge entfernt wurde.

Nach 10 bis 25 Tagen werden die überlebenden und teilungsaktiven Zellen auf einem Selektionsmedium kultiviert. Das Medium sollte zur osmotischen Stabilisierung der Protoplastenkalli vorteilhaft 6 bis 10 % Zucker, insbesondere Saccharose enthalten. Je älter die Protoplastenkultur ist, umso niedriger kann der Zuckergehalt sein. Beispielsweise ist für eine 15 bis 20 Tage alte Protoplastenkultur ein Medium mit ca. 9 % Zucker geeignet. Das Selektionsmedium ist im wesentlichen wie das oben beschriebene Wachstumsmedium zusammengesetzt und enthält ein Selektionsagens, wie z.B. Phosphinothricin. Die Selektion wird beispielsweise wie in EP 0 290 987 beschrieben, durchgeführt.

Auf dem beschriebenen Nährmedium entwickeln sich aus den transformierten Protoplasten das Gen exprimierende Zellcluster, die dann zu makroskopisch sichtbaren Kalli heranwachsen. 14 bis 28 Tage nach Beginn der Selektion werden die Kalli auf frisches, vorzugsweise hormonfreies Selektionsmedium übertragen, um die Embryoreifung zu ermöglichen. Die gebildeten transgenen Embryonen können bei nachfolgender Kultivierung, mit oder auch ohne Selektionsagens im Medium, Blätter und Wurzeln ausdifferenzieren.

Sobald die in vitro Pflanzen eine Größe von 4 bis 8 cm erreicht haben, werden sie in Erde gepflanzt und in einer Klimakammer oder im Gewächshaus zu blühenden Pflanzen aufgezogen. Die fertilen, transgenen Maispflanzen können sowohl durch Selbstbestäubung als auch durch Fremdbestäubung, d.h. durch Kreuzung mit Elite-Inzuchtlinien vermehrt werden.

Die Erfindung betrifft außerdem ein Verfahren zum Schutz von monokotylen Pflanzen, durch selektive Vernichtung von Unkraut mit Phosphinothricin oder Phosphinothricinalanylalanin, indem Anbauflächen mit Pflanzen, die aus dem erfindungsgemäßen Verfahren erhältlich sind und die das Phosphinothricinacetyltransferasegen tragen, sowie mit deren Nachkommen bepflanzt werden.

In den folgenden Beispielen wird die Erfindung weitergehend beschrieben.

### Beispiele

### Protoplastenisolierungsmedium (100 ml)

| | |
|---|---|
| Cellulase Onozuka R S (Meiji Seika, Japan) | 800 mg |
| Pectolyase Y 23 | 40 mg |
| KNO₃ | 200 mg |
| KH₂PO₄ | 136 mg |
| K₂HPO₄ | 47 mg |
| CaCl₂·2H₂O | 147 mg |
| MgSO₄·7H₂O | 250 mg |
| Bovine serum albumin (BSA) | 20 mg |
| Glucose | 4000 mg |
| Fructose | 4000 mg |
| Saccharose | 1000 mg |
| pH | 5,8 |
| Osmolarität | 660 mOsm. |

Protoplastenwaschlösung 1: wie oben aber ohne Cellulase, Pectolyase und BSA

### Transformationspuffer

| | | |
|---|---|---|
| a) | Glucose | 0,5 M |
| | MES | 0,1 % |
| | MgCl₂·6H₂O | 25 mM |
| | pH | 5,8 |
| | auf 600 mOsm. einstellen | |

| | | |
|---|---|---|
| b) | PEG 6000-Lösung | |
| | Glucose | 0,5 M |
| | MgCl₂·6H₂O | 100 mM |
| | Hepes | 20 mM |
| | pH | 6,5 |

Dem obigen Puffer unter b) wird PEG 6000 kurz vor Gebrauch der Lösung zugesetzt (40 Gew.-% PEG). Die PEG-Lösung wird durch ein 0,45 µm Sterilfilter filtriert.

### W 5 Lösung

| | |
|---|---|
| CaCl₂ | 125 mM |
| NaCl | 150 mM |
| KCl | 5 mM |
| Glucose | 50 mM |

### Protoplasten-Kulturmedium (Angaben in mg/l)

| | |
|---|---|
| KNO₃ | 3000 |
| (NH₄)₂SO₄ | 500 |
| MgSO₄·7H₂O | 350 |
| KH₂PO₄ | 400 |
| CaCl₂·2H₂O | 300 |

Fe-EDTA und Spurenelemente wie im Murashige-Skoog-Medium (Physiol. Plant, 15, 473 (1962)).

| | |
|---|---|
| m-Inosit | 100 |
| Thiamin HCl | 1,0 |
| Nicotinsäureamid | 0,5 |
| Pyridoxin HCl | 0,5 |
| Glycin | 2,0 |
| Glucuronsäure | 750 |
| Galacturonsäure | 750 |
| Galactose | 500 |
| Maltose | 500 |
| Glucose | 36.000 |
| Fructose | 36.000 |
| Saccharose | 30.000 |
| Asparagin | 500 |
| Glutamin | 100 |
| Prolin | 300 |
| Caseinhydrolysat | 500 |
| 2,4-Dichlorphenoxyessigsäure (2,4-D) | 0,5 |
| pH | 5,8 |
| Osmolarität | 600 mOsm |

### Starter- und Erhaltungsmedium für somatische Kulturen

Das modifizierte MS-Medium (Murashige, T., und Skoog, F., Physiol. Plant., 15 (1962) 473-497) nach Green, C.E., und Phillips, R.L., Crop Sci., 15 (1975) 417-421, aber mit den folgenden Modifikationen und Zusätzen (Endkonzentration in mg/l): Bacto-trypton 500 mg, L-Asparagin 150 mg.

### Startermedium für Antherenkulturen

N6-Makroelemente, Glycin und Vitamine nach Chu et al., Scientia Sinica, 18 (1975) 659, MS-Mikroelemente, EDTA und FeSO₄ . 7 H₂O nach Murashige, T., und Skoog, F., Physiolog. Plant, 15 (1962) 473, und die folgenden Bestandteile (pro Liter): L-Prolin 100 mg, Bacto-trypton (BT) 600 mg, Saccharose 120 g, Benzyladenin 1 mg, Naphthylessigsäure 1 mg, 2,4-Dichlorphenoxyessigsäure (2,4-D) 2 mg, Aktivkohle 5 g. Der pH-Wert wird vor dem Autoklavieren und der Zugabe von Aktivkohle mit KOH auf 5,8 ± 0,05 eingestellt. Das Medium wird mit 5 g/l Agarose verfestigt.

### Erhaltungsmedium für Haploidkulturen

Makroelemente, Mikroelemente, EDTA, FeSO₄ . 7 H₂O, Aminosäuren und Vitamine wie im Antherenkultur-Startermedium. Die geänderten Bestandteile sind (pro Liter): 2,4-D 0,5 mg, Saccharose 30 g, Agar 8 g. Der pH-Wert wird vor dem Autoklavieren auf 5,8 ± 0,05 eingestellt.

### Suspensionslkultur-(N₆M-)Medium

Dasselbe wie das Haploidkultur-Erhaltungsmedium ohne Agar.

### Beispiel 1:

Herstellung des opaken somatischen Kultursynthetikums MR Syn So₂o₂/87 und der überwiegend frühembryogenen Kultur OK281

Pflanzen verschiedener Maisgenotypen wurden auf dem Feld herangezogen. Drei bis vier Pflanzen wurden selbstbestäubt, und die befruchteten Kolben wurden 12-14 Tage nach der Bestäubung geerntet. Nach Entfernung von Schalen und Fäden wurden die Kolben 10 Minuten lang in 0,15% Natriumhypochloritlösung mit 0,5% Haushaltsreiniger oberflächensterilisiert und anschließend 3 mal mit sterilem entionisiertem Wasser gespült. Zehn Embryos pro Kolben wurden dann aseptisch abgetrennt und mit ihrer flachen Sproßknospen- und Keimwurzelseite nach unten auf 50 ml verfestigtes (0,8% Agar) somatisches Kulturstarter- und -erhaltungsmedium in 10 x 2 cm Petrischalen gelegt. Die Embryokulturen wurden für 30-60 Tage unter Beleuchtung bei 25 ± 2°C inkubiert. Nach der Untersuchung wurden die embryogenen Kulturen in monatlichen Abständen auf frisches Medium überführt und auf die Fähigkeit zur Pflanzenregeneration auf Pflanzenregenerationsmedium untersucht. Von den geprüften Genotypen bildeten die Inzuchtlinien A188, GK3/H und W64A, der Genstamm Mangelsdorf-Tester (MT) und eine lokale "white flint"-Varietät (WFLV) unter den beschriebenen Bedingungen regenerierbare Kulturen. Diese Genotypen bildeten jedoch überwiegend spätembryogene Kulturen, die in der Literatur zur Maisgewebekultur häufig als Typ-I-Kultur bezeichnet wird. Ihr Regenerationspotential nahm nach aufeinanderfolgenden Transfers auf frisches Medium allmählich ab, und nach 6monatiger Erhaltung waren nur vereinzelte kleine regenerierbare Abschnitte vorhanden. Wenn Hybride aus zwei regenerierbaren Genotypen gebildet und ihre Embryokulturen in derselben Weise geprüft wurden, wurde eine Verbesserung der Fortpflanzungsrate und der Regenerierbarkeit beobachtet. Die Kultur aus dem Hybriden A188 x W64A zum Beispiel wurde 3 Jahre lang ohne den Verlust des Regenerationspotentials erhalten. Um die weitere Kombination der möglichen vorteilhaften Gene aus den ursprünglichen Genotypen zu fördern, wurde ein Kreuzungsprogramm über mehrere Generationen gestartet. Zwei opake (o₂o₂) Quellen, die entweder unter diesen Bedingungen nicht regenerierbar waren (Oh 43o₂o₂) oder nicht auf ihre Fähigkeit zur Regeneration aus Gewebekulturen geprüft wurden (H Temp Ao₂o₂), wurden einbezogen. Um das opake Gen mit den für das regenerierbare Merkmal verantwortlichen Genen zu kombinieren, mußten zuerst die opaken Quellengenotypen mit den regenerierbaren Genotypen gekreuzt werden. Deshalb wurden diese Genotypen gepflanzt, kultiviert, und ihre Kolben wurden vor der Entwicklung der Fäden isoliert. Die isolierten Kolben der regenerierbaren Genotypen wurden mit Pollen von Genotypen, die das opake Gen trugen, kreuzbestäubt. Somit wurden die Einzelkreuzungshybriden (SC1 - SC10) durch Kreuzung von A188, GK3/H, W64A, MT und WFLV mit Oh43o₂o₂ und H Temp Ao₂o₂ gewonnen. Die Körner dieser 10 verschiedenen Einzelkreuzungen wurden geerntet und wie zuvor beschrieben gepflanzt, kultiviert und für die zweite Serie der Kreuzbestäubungen isoliert, um die Körner der Doppelkreuzungshybriden (DC1 - DC5) aus den Kreuzungen SC 1 x SC7, SC2 x SC8, SC3 x SC9, SC4 x SC10 bzw. SC5 x SC6 zu gewinnen. Aus der ersten und zweiten Bestäubungsserie wurde nur ein geernteter Kolben von jeder Kreuzbestäubung für die weitere Vermehrung verwendet. Von den geernteten DC-Körnern wurden homozygote o₂o₂-Körner anhand ihres Phänotyps ausgewählt und gepflanzt. Zur Blütezeit wurde in einer dritten Bestäubungsserie jeder DC-Hybride (DC1 bis DC5) mit allen anderen gekreuzt, um 4 geerntete Kolben von jeder der 20 Kreuzkombinationen zu erhalten. Aus den resultierenden 80 Kolben wurden 10 Körner pro Kolben gequollen, gepflanzt und zufällig geschwisterbestäubt, um die Möglichkeit zur Rekombination der gewünschten Gene zu schaffen. Die einhundert besten Kolben wurden aus dem geernteten geschwistergekreuzten Pflanzenmaterial entnommen, 10 Körner von jedem Kolben wurden gequollen und als somatisches Ausgangskultursynthetikum betrachtet (MR Syn So₂o₂/87). Das gewonnene Synthetikum wurde gepflanzt, und die blühenden Pflanzen wurden selbstbestäubt. Von jedem der 100 selbstbestäubten Kolben wurden 10 unreife Embryos in der beschriebenen Weise plattiert. Etwa 30% der kultivierten Embryos (286 von 950) bildeten regenerierbare Kulturen, wobei die meisten spätembryogene Kulturen waren. Von den gewonnenen spontan frühembryogenen Kulturen, die in der einschlägigen Literatur häufig als Typ-IIoder "krümelige" embryogene Kultur bezeichnet werden, wurde die Kultur OK281 aufgrund ihres kräftigen Wachstums, der Abwesenheit von Nekrosen, der leichten Regenerierbarkeit zu Pflanzen und der Fruchtbarkeit der regenerierten Pflanzen ausgewählt.

### Beispiel 2:

Herstellung des Antherenkultursynthetikums MR Syn A/85 und der frühembryogenen haploiden Kultur H 229

Verschiedene Maisgenotypen wurden gepflanzt und bis zum entsprechenden physiologischen Stadium kultiviert, um Fahnen mit geöffneten obersten Blättern zu gewinnen. Die äußeren Blätter wurden aufgeklappt und verworfen. Die noch im Deckquirl verbliebenen Fahnen wurden in Kunststoffbeutel gegeben und bei einer Temperatur von 4 bis 8°C im Kühlschrank für 10-30 Tage kältebehandelt. Nach dieser Behandlung wurden die Deckblätter entfernt, und die Fahnenabschnitte mit Antheren, die spätmononukleäre Mikrosporen trugen, wurden mittels mikroskopischer Untersuchung von Antheren ausgewählt, die in 2%iger wässriger Orcein-Lösung zerstoßen wurden. Die Einzelblüten mit den gewünschten Mikrosporen wurden 10 Minuten lang in 0,1% Natriumhypochloritlösung mit 0,5% Haushaltsreiniger oberflächensterilisiert und anschließend 3 x 10 Minuten lang in entionisiertem sterilem Wasser gespült. Von den oberflächensterilisierten Einzelblüten wurden etwa 100 Antheren abgetrennt und auf 50 ml Antherenkultur-Startermedium in 10 x 2 cm Petrischalen aus Glas gegeben. Die plattierten Antheren wurden im Dunkeln für 40-90 Tage bei 25°C inkubiert. Zwei lokale Varietäten, Golden Glow (GG) und eine lokale "white dent"-Varietät (WDLV), bildeten zu einem geringen Anteil (unter 1%) haploide Calli aus den kultivierten Antheren. Keiner davon regenerierte Pflanzen auf dem Regenerationsmedium. Die beiden lokalen Varietäten (GG und WDLV) wurden gekreuzt, hybride Körner wurden gepflanzt, und Antheren der resultierenden F1-Pflanzen wurden in derselben Weise geprüft. Von den gewonnenen haploiden Embryoiden konnte eine Kultur für die mehrfache Pflanzenregeneration während eines Zeitraums von einem Jahr verwendet werden, bevor sie ihr Regenerationspotential verlor. Um das Embryoidbildungspotential, die Pflanzenregenerationsfähigkeit und die für das Überleben und Wachstum der in Erde verpflanzten Regeneranten notwendigen Pflanzenmerkmale zu kombinieren, wurden zehn Pflanzen des Hybriden der beiden lokalen Varietäten mit einer Pflanzenpopulation kreuzbestäubt, die durch Kreuzhybridisierung von zwei unabhängigen Hybriden [GK3/H x MT und (A188 x A619) x BMSC], die jeweils in der Antherenkultur ansprachen, gewonnen worden war. Zehn Körner der geernteten Kolben wurden gequollen, im Feld gepflanzt, und die auf Fehlen von unerwünschten agronomischen Merkmalen (Bestockung, Maisbrand, Lagern) selektierten Pflanzen wurden geschwistergekreuzt. Nach der Ernte wurden die 30 besten Kolben behalten und für die Quellung einer gleichen Anzahl von Körnern pro Kolben verwendet. Diese stellten das Antherenkultursynthetikum MR Syn A/85 dar. Eine der gequollenen Mengen des Synthetikums wurde gepflanzt, und Fahnen wurden in der beschriebenen Weise gesammelt und verarbeitet. Bei den kultivierten Antheren wurde eine Embryoidbildung von 0 bis etwa 20% pro Petrischale beobachtet. Von mehr als 300 haploiden Embryoiden, die von verschiedenen Fahnen des MS Syn A/85 stammten, entwickelten sich nach Überführung aus dem Startermedium in Haploid-Erhaltungsmedium etwa 20% zu regenerierbaren embryogenen Kulturen. Aus den regenerierbaren Kulturen wurde die Kultur H 229 aufgrund ihres überwiegend frühembryogenen Kallus-Phänotyps, ihrer raschen Fortpflanzungsrate, ihrer Pflanzenregenerationsfähigkeit nach Langzeit-Oberflächen- und Suspensionskultur (mehr als zwei Jahre bis zur Verwendung für einen weiteren Kreuzungszyklus) sowie ihrer Fähigkeit, sich zu teilen und Kolonien zu bilden und Pflanzen aus Protoplasten zu regenerieren, ausgewählt.

### Beispiel 3:

Herstellung der hochembryogenen Kultur DSM 6009.

Zwei zuvor selektierte Genotypen, die überwiegend frühembryogene Kulturen aus Embryos oder Antheren entwickeln, wurden kreuzbestäubt, um die erwünschten Merkmale beider Genotypen zu kombinieren. Weibliche Blüten der regenerierten H229-Pflanzen wurden 30 Monate nach dem Ansetzen mit dem Pollen von OK281-Pflanzen, die 10 Monate nach dem Ansetzen regeneriert wurden, bestäubt Die gewonnenen unreifen Hybridembryos wurden auf somatischem Kulturstarter- und Erhaltungsmedium sowie auf Pflanzenregenerationsmedium inkubiert. Einer der Embryos, der eine frühembryogene Kultur auf dem Pflanzenregenerationsmedium bildete, DSM 6009 (HE 89), wurde ausgewählt, erhalten und zum Anlegen von Suspensionskulturen verwendet. Lediglich zwei Monate nach Inokulation des Spenderhybridembryos (HL29 x OK281) wurde die erste erfolgreiche Protoplastenkultur gestartet, die zur Ausbildung von fruchtbaren protoplastenstämmigen Pflanzen führte. DSM 6009 hat alle gewünschten Eigenschaften seiner Elterngeneration und ist ihnen in vielen der oben aufgeführten Gewebekulturmerkmale überlegen.

### Beispiel 4:

### Untersuchung des Suspensionskulturmediums N6M auf die Retention der Regenerierbarkeit in den ausgewählten Maisgewebekulturen

Seit der Entwicklung des Suspensionskulturmediums N6M sind 3 unabhängig selektierte regenerierbare Genotypen (einschließlich früh- und spätembryogene, somatische und haploide Kulturen) für die Dauer von mehr als zwei Jahren in Suspensionskultur auf ihre regenerativen und Fruchtbarkeitseigenschaften geprüft worden. Die somatische, überwiegend spätembryogene Kultur 4C1, die somatische, spätembryogene Kultur C2-A und die haploide, überwiegend frühembryogene Kultur H 229 wurden in N6M-Suspensionskulturmedium gehalten. Obwohl Suspensionskulturen eine schnellere Fortpflanzungsrate zeigen, war die Pflanzenregenerationsfähigkeit dieser selektierten Genotypen bei aus Suspensionskulturen plattierten Embryoiden besser als bei Embryoiden aus Agar-Oberflächenkulturen, die in langen Abständen (etwa 2 Monate) passagiert wurden. In allen drei Fällen konnten nach zwei Jahren der Erhaltung in N6M-Suspensionskultur fruchtbare Pflanzen gewonnen werden. Ähnliche Beobachtungen wurden mit anderen Kulturen nach einem Jahr der Erhaltung in dieser Suspensionskultur gemacht. Deshalb wird das N6M-Snspensionskulturmedium für die Erhaltung der Kulturen vor der Isolierung der Protoplasten bevorzugt.

### Anlegen und Erhaltung der HE/89-Suspensionskultur

Die weniger als 1 mm großen Embryos (H 229 x OK 281), die aus einer Gewächshauspflanze 12 Tage nach der Bestäubung gewonnen wurden, wurden auf N6M ohne 2,4-D für 3 Wochen inkubiert, gefolgt von zwei Subkulturen auf N6M- und MSG-Medium in Abständen von 10-14 Tagen, bevor die Suspensionskultur in N6M gestartet wird. Zur regelmäßigen Erhaltung werden 4 g Zellen verwendet, um einen neuen Kuhurzyldus von 5-7 Tagen in 50 ml N6M-Medium in 100-ml-Erlenmeyer-Kolben zu starten. Alle Kulturen werden unter kontinuierlicher oder periodischer Beleuchtung durch Fluoreszenzröhren bei 22-25°C gehalten.

### Definitionen

### H 229:

Regenerierbare haploide Gewebekultur und Genotyp von Zea mays (L.), gewonnen durch Antherenkultur aus dem Antherenkultursynthetikum MS SynA/85 und hergestellt durch sukzessive Kreuz- und Geschwisterbestäubungen unter Einbeziehung der folgenden Genotypen: A188, A629, GK 3/H (Inzuchtlinien), Black Mexican Sweet Corn (BMSC), Mangelsdorf-Tester (MT) (Genstämme), "white dent" lokale Varietät (WDLV) und Golden Glow (GG) (lokale Varietäten). Die H-229-Kultur ist überwiegend frühembryogen und bildet leicht Suspensionskulturen, während sich die Suspensionskulturprotoplasten unter definierten Bedingungen zu Pflanzen entwickeln. Die aus der H-229-Gewebekultur gewonnenen H-229-Pflanzen sind weiblich-fiuchtbar und bilden nach Bestäubung mit lebensfähigen Pollenkömern Samen.

### OK 281:

Regenerierbare, überwiegend frühembryogene somatische Gewebekultur und Genotyp von Zea mays (L.), gewonnen durch unreife Embryonenkultur aus dem MR Syn So₂o₂/87 und hergestellt durch sukzessive Kreuz-, Geschwister- und Selbstbestäubungen unter Einbeziehung der folgenden Genotypen: A188, GK 3/H, W 64 A, Oh 43o₂o₂ (Inzuchtlinien), MT (Genstamm), "white flint" lokale Varietät (WFLV) (lokale Varietät) und H Temp Ao₂o₂ (o₂o₂-Synthetikum).

Die aus den OK-281-Kulturen gewonnenen OK-281-Pflanzen bilden Samen durch Selbst-, Geschwister- oder Kreuzbestäubung unter geeigneten Bedingungen. Der Genotyp OK 281 ist homozygot für das rezessive Opakgen (o₂o₂).

### DSM 6009 (HE/89):

Regenerierbare, hochembryogene, überwiegend frühembryogene somatische Kultur und Genotyp von Zea mays (L.), gewonnen durch unreife Embryonenkultur nach Kreuzbestäubung zwischen den beiden zuvor selektierten Genotypen H 229 und OK 281. Die Genotypen H 229 und OK 281 wurden für 30 bzw. 10 Monate in Kultur gehalten, bevor Pflanzen für Kreuzbestäubungen daraus regeneriert wurden. Der Genotyp DSM 6009 ist aufgrund seiner Abstammung von Vorfahren, die wegen ihrer Gewebekulturmerkmale ausgewählt wurden, besonders angepaßt an die Anforderungen der In-vitro-Kultur. Somit besitzt DSM 6009 eine einzigartige Kombination der folgenden Merkmale:
1. Bildung von hormonautotrophem embryogenen Kallus, der gut auf normalen Pflanzenkulturmedien wächst.
2. Reproduzierbare Bildung von kallusartigen frühembryogenen (Typ-II-Kallus)-Kulturen aus reifen somatischen Embryos auf auxinhaltigen Medien.
3. Zuverlässige langfristige Pflanzenregenerationsfähigkeit.
4. Reproduzierbare Umwandlung von kallusartigen frühembryogenen (Typ-II-Kallus)-Kulturen zu Suspensionskulturen.
5. Üppige Protoplastenfreisetzung einen Monat nach Überführung des Kallus in Flüssigkultur.
6. Hohe Regenerationsrate mit voll fruchtbaren Pflanzen aus Kallus, Suspensionen und Protoplasten.

### Früh- und spätembryogene Kulturen:

Eine besondere Art von Mais-(Zea-mays-L.)-Gewebekulturen, bei denen eine rasche Vermehrungsrate früher Stadien von somatischen Embryos beobachtet wird. Diese Embryoide reifen nicht zu deutlich sichtbaren Embryoiden heran, die charakterisiert sind durch ihre Größe und glatte, scutellumartige Strukturen, die Blatt- und Wurzelanlagen tragen. Vielmehr haben die frühembryogenen Kulturen das Aussehen einer homogenen Kalluskultur. Dieser Kulturtyp wird in der einschlägigen Literatur als Typ-II- oder "krümelige" embryogene Kalluskultur bezeichnet. Die frühembryogenen Kulturen entwickeln sich vor der Pflanzenregeneration zu spätembryogenen Kulturen. In den spätembryogenen Kulturen sind die Embryoide vergrößert mit gut entwickelten scutellumartigen Körpern, grünen Flecken bzw. Blatt und Triebanlagen sowie mit Wurzelanlagen oder kleinen Wurzeln. In DSM-6009-Kulturen sind beide Kallusarten leicht zugänglich und je nach Kulturbedingungen untereinander austauschbar.

### 5. Herstellung von Protoplasten der Zellinie DSM 6009

### Protoplastenisolierung

2-4 Tage, vorzugsweise 3 Tage nach dem letzten Mediumswechsel der Suspensionskultur wird das Flüssigmedium abgesaugt und die zurückbleibenden Zellen mit 50 ml Protoplastenwaschlösung 1 gespült und nochmals trockengesaugt. Zu jeweils 2 g der geernteten Zellmasse wird 10 ml Protoplastenisolierungsmedium gegeben. Die resuspendierten Zellen und Zellaggregate werden bei 27 ± 2°C unter leichtem Schütteln (30 bis 40 rpm) 4 bis 6 h im Dunkeln inkubiert.

### Protoplastenreinigung

Sobald die Freisetzung von mindestens 1 Mio. Protoplasten/ml erfolgt ist (mikroskopische Beobachtung), wird die Suspension durch ein Edelstahl- und Nylonsieb von 200 bzw.45 µm Maschenwerte gesiebt. Die Kombination eines 100 µm und eines 60 µm Siebs ermöglicht die Abtrennung der Zellaggregate genauso gut. Das protoplastenhaltige Filtrat wird mikroskopisch beurteilt. Üblicherweise enthält es 98-99 % Protoplasten. Der Rest sind unverdaute Einzelzellen. Protoplastenpräparationen mit diesem Reinheitsgrad werden ohne zusätzliche Gradientenzentrifugation für Transformationsexperimente verwendet. Durch Zentrifugation, 1000 UpM im Ausschwingrotor (100 x g, 3 min) werden die Protoplasten sedimentiert. Der Überstand wird verworfen und die Protoplasten in Waschlösung 1 resuspendiert. Die Zentrifugation wird wiederholt und die Protoplasten danach im Transformationspuffer resuspendiert.

### 6. Klonierung des Phosphinothricinacetyltransferasegens

Zur Tranformation wird das unten angegebene synthetische Phosphinothricin-acetyltransferasegen unter der Kontrolle des Promotors des 35 S-Transcripts des Cauliflower-Mosaic-Virus (vgl. Sequenzausdruck unten; das auf S. 13 abgebildete Phosphinothricinacetyltransferasestrukturgen ist in die Sal I-Schnittstelle des auf S. 12 aufgeführten pDH51 kloniert), bzw. das synthetische Gen aus der Europäischen Patentanmeldung EP 0 275 957 verwendet. Das chimäre Gen bestehend aus 35 S-Promotor Strukturgen und 35 S-Terminator ist in die EcoRI-site des Polylinkers von pUC18 integriert.

Es wird in der Mehrzahl der Experimente ungeschnittenes pUC18-35S-Acetyltransferase-Plasmid verwendet. In einzelnen Experimenten wird das Plasmid mit EcoRI gespalten und die Mischung von linearisiertem pUC18-Plasmid und dem 1,3 Kb-35S-Acetyltransferase-Insert zur Transformation verwendet.

### Sequenz Phosphinothricinacetyltransferasegen:

EcoRI
GAATTCCCATGGAGTCAAAGAATCAAATAGAGGACCTAACAGAACTCGCCGTAAAGACTGGCGAACAGTTCA
CTTAAGGGTACCTCAGTTTCTTAGTTTATCTCCTGGATTGTCTTGAGCGGCATTTCTGACCGCTTGTCAAGT
12 24 36 48 60 72

TACAGAGTCTCTTACGACTCAATGACAAGAAGAAAATCTTCGTCAACATGGTGGAGCACGACACGCTTGTCT
ATGTCTCAGAGAATGCTGAGTTACTGTTCTTCTTTTAGAAGCAGTTGTACCACCTCGTGCTGTGCGAACAGA
84 96 108 120 132 144

ACTCCAAAAATATCAAAGATACAGTCTCAGAAGACCAAAGGGCAATTGAGACTTTTCAACAAAGGGTAATAT
TGAGGTTTTTATAGTTTCTATGTCAGAGTCTTCTGGTTTCCCGTTAACTCTGAAAAGTTGTTTCCCATTATA
156 168 180 192 204 216

CCGGAAACCTCCTCGGATTCCATTGCCCAGCTATCTGTCACTTTATTGTGAAGATAGTGGAAAAGGAAGGTG
GGCCTTTGGAGGAGCCTAAGGTAACGGGTCGATAGACAGTGAAATAACACTTCTATCACCTTTTCCTTCCAC
228 240 252 264 276 288

GCTCCTACAAATGCCATCATTGCGATAAAGGAAAGGCCATCGTTGAAGATGCCTCTGCCGACAGTGGTCCCA
CGAGGATGTTTACGGTAGTAACGCTATTTCCTTTCCGGTAGCAACTTCTACGGAGACGGCTGTCACCAGGGT
300 312 324 336 348 360

AAGATGGACCCCCACCCACGAGGAGCATCGTGGAAAAAGAAGACGTTCCAACCACGTCTTCAAAGCAAGTGG
TTCTACCTGGGGGTGGGTGCTCCTCGTAGCACCTTTTTCTTCTGCAAGGTTGGTGCAGAAGTTTCGTTCACC
372 384 396 408 420 432

ATTGATGTGATATCTCCACTGACGTAAGGGATGACGCACAATCCCACTATCCTTCGCAAGACCCTTCCTCTA
TAACTACACTATAGAGGTGACTGCATTCCCTACTGCGTGTTAGGGTGATAGGAAGCGTTCTGGGAAGGAGAT
444 456 468 480 492 504

SalI
TATAAGGAAGTTCATTTCATTTGGAGAGGACAGGGTACCCGGGGATCCTCTAGAGTCGACCTGCAGGCATGC
ATATTCCTTCAAGTAAAGTAAACCTCTCCTGTCCCATGGGCCCCTAGGAGATCTCAGCTGGACGTCCGTACG
516 528 540 552 564 576

CGCTGAAATCACCAGTCTCTCTCTACAAATCTATCTCTCTCTATAATAATGTGTGAGTAGTTCCCAGATAAG
GCGACTTTAGTGGTCAGAGAGAGATGTTTAGATAGAGAGAGATATTATTACACACTCATCAAGGGTCTATTC
588 600 612 624 636 648

GGAATTAGGGTTCTTATAGGGTTTCGCTCATGTGTTGAGCATATAAGAAACCCTTAGTATGTATTTGTATTT
CCTTAATCCCAAGAATATCCCAAAGCGAGTACACAACTCGTATATTCTTTGGGAATCATACATAAACATAAA
660 672 684 696 708 720

EcoRI
GTAAAATACTTCTATCAATAAAATTTCTAATTCCTAAAACCAAAATCCAGTGGGTACCGAGCTCGAATTCAA
CATTTTATGAAGATAGTTATTTTAAAGATTAAGGATTTTGGTTTTAGGTCACCCATGGCTCGAGCTTAAGTT
732 744 756 768 780 792

G
C

- Val Asp Met Ser Pro Glu Arg Arg Pro Val Glu Ile Arg Pro Ala Thr Ala
SalI
GTC GAC ATG TCT CCG GAG AGG AGA CCA GTT GAG ATT AGG CCA GCT ACA GCA
CAG CTG TAC AGA GGC CTC TCC TCT GGT CAA CTC TAA TCC GGT CGA TGT CGT
12 24 36 48

Ala Asp Met Ala Ala Val Cys Asp Ile Val Asn His Tyr Ile Glu Thr Ser
GCT GAT ATG GCC GCG GTT TGT GAT ATC GTT AAC CAT TAC ATT GAG ACG TCT
CGA CTA TAC CGG CGC CAA ACA CTA TAG CAA TTG GTA ATG TAA CTC TGC AGA
63 75 87 99

Thr Val Asn Phe Arg Thr Glu Pro Gln Thr Pro Gln Glu Trp Ile Asp Asp
ACA GTG AAC TTT AGG ACA GAG CCA CAA ACA CCA CAA GAG TGG ATT GAT GAT
TGT CAC TTG AAA TCC TGT CTC GGT GTT TGT GGT GTT CTC ACC TAA CTA CTA
114 126 138 150

Leu Glu Arg Leu Gln Asp Arg Tyr Pro Trp Leu Val Ala Glu Val Glu Gly
CTA GAG AGG TTG CAA GAT AGA TAC CCT TGG TTG GTT GCT GAG GTT GAG GGT
GAT CTC TCC AAC GTT CTA TCT ATG GGA ACC AAC CAA CGA CTC CAA CTC CCA
165 177 189 201

Val Val Ala Gly Ile Ala Tyr Ala Gly Pro Trp Lys Ala Arg Asn Ala Tyr
GTT GTG GCT GGT ATT GCT TAC GCT GGG CCC TGG AAG GCT AGG AAC GCT TAC
CAA CAC CGA CCA TAA CGA ATG CGA CCC GGG ACC TTC CGA TCC TTG CGA ATG
216 228 240 252

Asp Trp Thr Val Glu Ser Thr Val Tyr Val Ser His Arg His Gln Arg Leu
GAT TGG ACA GTT GAG AGT ACT GTT TAC GTG TCA CAT AGG CAT CAA AGG TTG
CTA ACC TGT CAA CTC TCA TGA CAA ATG CAC AGT GTA TCC GTA GTT TCC AAC
267 279 291 303

Gly Leu Gly Ser Thr Leu Tyr Thr His Leu Leu Lys Ser Met Glu Ala Gln
GGC CTA GGA TCC ACA TTG TAC ACA CAT TTG CTT AAG TCT ATG GAG GCG CAA
CCG GAT CCT AGG TGT AAC ATG TGT GTA AAC GAA TTC AGA TAC CTC CGC GTT
318 330 342 354

Gly Phe Lys Ser Val Val Ala Val Ile Gly Leu Pro Asn Asp Pro Ser Val
GGT TTT AAG TCT GTG GTT GCT GTT ATA GGC CTT CCA AAC GAT CCA TCT GTT
CCA AAA TTC AGA CAC CAA CGA CAA TAT CCG GAA GGT TTG CTA GGT AGA CAA
369 381 393 405

Arg Leu His Glu Ala Leu Gly Tyr Thr Ala Arg Gly Thr Leu Arg Ala Ala
AGG TTG CAT GAG GCT TTG GGA TAC ACA GCC CGG GGT ACA TTG CGC GCA GCT
TCC AAC GTA CTC CGA AAC CCT ATG TGT CGG GCC CCA TGT AAC GCG CGT CGA
420 432 444 456

Gly Tyr Lys His Gly Gly Trp His Asp Val Gly Phe Trp Gln Arg Asp Phe
GGA TAC AAG CAT GGT GGA TGG CAT GAT GTT GGT TTT TGG CAA AGG GAT TTT
CCT ATG TTC GTA CCA CCT ACC GTA CTA CAA CCA AAA ACC GTT TCC CTA AAA
471 483 495 507

Glu Leu Pro Ala Pro Pro Arg Pro Val Arg Pro Val Thr Gln Ile @@@ Val
SalI
GAG TTG CCA GCT CCT CCA AGG CCA GTT AGG CCA GTT ACC CAG ATC TGA GTC
CTC AAC GGT CGA GGA GGT TCC GGT CAA TCC GGT CAA TGG GTC TAG ACT CAG
522 534 546 558

Asp
GAC
CTG

### 7. Protoplastentransformation

Die in Transformationspuffer resuspendierten Protoplasten werden bei einem Titer von 0,5-1,10⁶ Protoplasten/ml in 10 ml Portionen in 50 ml Polyallomer-Röhrchen eingefüllt. Die zur Transformation verwendete DNA wird in Tris-EDTA (TE) Puffer gelöst. Pro ml Protoplastensuspension werden 20 µg Plasmid-DNA zugegeben. Nach der DNA-Zugabe wird die Protoplasten-Suspension vorsichtig geschüttelt, um die DNA homogen in der Lösung zu verteilen. Sofort danach wird tropfenweise 5 ml PEG-Lösung zugetropft.

Durch vorsichtiges Schwenken der Röhrchen wird die PEG-Lösung homogen verteilt. Danach werden nochmals 5 ml PEG-Lösung zugegeben und das homogene Durchmischen wiederholt. Die Protoplasten verbleiben 20 min in der PEG-Lösung bei 25 ± 2°C. Danach werden die Protoplasten durch 3 minütiges Zentrifugieren (100 g =̂ 1000 Upm) sedimentiert. Der Überstand wird verworfen. Die Protoplasten werden durch vorsichtiges Schütteln in 20 ml W₅-Lösung gewaschen und danach erneut zentrifugiert. Danach werden sie in 20 ml Protoplastenkulturmedium resuspendiert, nochmals zentrifugiert und erneut in Kulturmedium resuspendiert. Der Titer wird auf 6-8 ·10⁵ Protoplasten/ml eingestellt und die Protoplasten in 3 ml Portionen in Petrischalen (φ 60 mm, Höhe 15 mm) kultiviert. Die mit Parafilm versiegelten Petrischalen werden bei 25 ± 2°C im Dunkeln aufgestellt.

### 8. Protoplastenkultur

Während der ersten 2-3 Wochen nach der Protoplastenisolierung und -Transformation werden die Protoplasten ohne Zugabe von frischem Medium kultiviert. Sobald sich die aus den Protoplasten regenerierten Zellen zu Zellaggregaten mit mehr als 20-50 Zellen entwickelt haben, wird 1 ml frisches Protoplastenkulturmedium zugegeben, das als Osmoticum Saccharose (90 g/l) enthält.

### 9. Selektion transformierter Maiszellen und Pflanzenregeneration

3-10 Tage nach der Zugabe von frischem Medium können die aus Protoplasten entstandenen Zellaggregate auf Agar-Medien mit 100 mg/l L-Phosphinothricin plattiert werden. N6-Medium mit den Vitaminen des Protoplastenkulturmediums, 90 g/l Saccharose und 1,0 mg/l 2,4-D ist ebenso geeignet wie ein analoges Medium beispielsweise mit den Makro- und Mikronährsalzen des MS-Mediums (Murashige + Skoog 1962).

Auf dem Selektivmedium können die aus stabil transformierten Protoplasten hervorgegangenen Kalli ungehindert weiterwachsen. Nach 3-5 Wochen, vorzugsweise 4 Wochen können die transgenen Kalli auf frisches Selektionsmedium transferiert werden, welches ebenfalls 100 mg/l L-Phosphinothricin enthält, das aber kein Auxin mehr enthält. Innerhalb von 3-5 Wochen differenzieren ca. 50 % der transgenen Maiskalli, die das L-Phosphinothricinacetyltransferase-Gen in ihr Genom integriert haben, auf diesem Medium in Gegenwart von L-Phosphinothricin erste Pflanzen.

### 10. Aufzucht transgener Regeneratpflanzen

Das embryogene transformierte Maisgewebe wird auf hormonfreiem N6-Medium (Chu C.C. et al., Sci. Sin. 16, 659, (1975)) in Gegenwart von 5·10⁻⁴ M L-Phosphinothricin kultiviert. Auf diesem Medium entwickeln sich Maisembryonen, die das Phosphinothricin-Acetyltransferase-Gen (PAT-Gen) hinreichend stark exprimieren, zu Pflanzen. Nicht transformierte Embryonen oder solche mit nur sehr schwacher PAT-Aktivität sterben ab. Sobald die Blätter der in vitro-Pflanzen eine Länge von 4-6 mm erreicht haben, können diese in Erde transferiert werden. Nach Abwaschen von Agrarresten an den Wurzeln werden die Pflanzen in ein Gemisch von Lehm, Sand, Vermiculit und Einheitserde im Verhältnis 3:1:1:1 gepflanzt und während der ersten 3 Tage nach dem Verpflanzen bei 90-100 % relativer Luftfeuchte an die Erdkultur adaptiert. Die Anzucht erfolgt in einer Klimakammer mit 14 h Lichtperiode ca. 25000 Lux in Pflanzenhöhe bei einer Tag/Nachttemperatur von 23 ± 17 ± 1°C. Die adaptierten Pflanzen werden bei einer Luftfeuchte von 65 ± 5 % kultiviert.

### 11. Expressionsnachweis des übertragenen Phosphinothricin-Acetyltransferase-Gens

3-4 Wochen nach dem Transfer in Erde werden transgene Regeneratpflanzen und als Kontrollen Regeneratpflanzen aus nicht mit DNA-behandelten Kalli mit Phosphinothricin in den handelsüblichen Formulierungen (Basta®), besprüht. Es wird eine Phosphinothricinmenge verwendet, die der Behandlung mit 2,0 kg Wirkstoff/ha entspricht. Das Herbizid wird in einer Wassermenge von 50 ml/m² (= 500 l/ha) ausgebracht. Von dieser Phosphinothricinmenge werden Pflanzen ohne PAT-Aktivität innerhalb von 7-10Tagen in Abhängigkeit vom Expressionsgrad des Gens entweder überhaupt nicht geschädigt oder, im Falle niedriger PAT-Aktivität, werden lokale Blattflecken sichtbar, die aber das Wachstum der behandelten Pflanzen nicht meßbar beeinflussen.

b) Jeweils 100 mg-Proben von Maiskallus und Blattgewebe werden in 1,5 ml Reaktionsgefäßen in flüssigem Stickstoff eingefroren und mit einem der Gefäßform angepaßten Mikropistill aus Edelstahl homogenisiert. Dazu wird 100 µl TRIS-EDTA-Puffer (50 mM Tris/2 mM EDTA pH 7,5) zugesetzt. Das Homogenat wird bei 12000 UpM eine Minute in einer Eppendorf-Zentrifuge zentrifugiert. Der klare Überstand wird abpipettiert und das Pellet verworfen. Es wird der Proteingehalt des Rohextraktes mittels des Bio-Rad Proteinassays ermittelt. Für den PAT-Test werden Aliquots der Rohextrakte genommen, die jeweils 20 µg Protein enthalten. Den Extrakten werden Acetyl-Co-A und 14C-L-Phosphinothricin zugesetzt. Die Endkonzentration im Inkubationsgemisch beträgt 1 mM für Acetyl-Co-A und 0,1 mM für 14C-L-Phosphinothricin. Die Proben werden kurz gemischt und bei 37°C 1 h inkubiert. Danach wird die Enzymreaktion durch Zugabe von ½ Vol 12 % Trichloressigsäure gestoppt, gut gemischt und 10 min auf Eis gestellt. Danach wird bei 12000 UpM 10 min zentrifugiert und der Überstand für die DC verwendet. Auf einer Dünnschichtplatte (Cellulose F254, Fa. Merck) werden je 6 µl der Inkubationsgemische aufgetragen, ebenso die Referenzsubstanzen 14C-Phosphinothricin und 14C-Acetylphosphinothricin. Als Laufmittel für die Dünnschichtchromatographie wird entweder n-Propanol: 25 % NH₃ (3:2) oder Pyridin:n-Butanol:Eisessig:Wasser (3:5:1:4) verwendet.

Die getrocknete Dünnschichtplatte wird in Kunststoffolie verpackt und in einer Röntgenfilmkassette exponiert. Auf dem entwickelten Röntgenfilm ist die PAT-Aktivität an der (nur im transgenen Gewebe vorkommenden) Acetylphosphinotricin-Bande erkennbar. Phosphinothricin und Acetylphosphinothricin sind dünnschichtchromatographisch in dem obengenannten Laufmitteln gut trennbar.

### 12. Vererbung des übertragenen Gens an die Nachkommen der Regeneratpflanzen

Pollen von Transformanten, die die Applikation einer Glufosinate Dosis von 2 kg ai/ha ohne Schädigung tolerieren und im PAT-Enzymtest eindeutig PAT-Aktivität aufweisen, wird zum Bestäuben von Narben diverser Inzuchtlinien verwendet, u.a. B73, LH119, LH82, LH51 bzw. Mo17. Da über die Pollen nur kerncodierte Gene weitervererbt werden, kann somit überprüft werden, ob das übertragene Gen in das Kerngenom integriert wurde. Nur dann kann eine Expression des Gens in den Nachkommen und ein den Mendelschen Gesetzen folgender Erbgang erwartet werden.

### a) Test an unreifen Embryonen

12-16 Tage nach der Bestäubung werden unter sterilen Bedingungen unreife Embryonen aus den Karyopsen präpariert. Die isolierten Embryonen werden auf hormonfreien MS bzw. N₆-Agar-Medium, das 9 % Saccharose und 100 mg/l L-Phosphinothricin-NH₄-Salz (Glufosinate-Ammonium) enthält, kultiviert. Die Embryonen werden in der Weise auf dem Medium kultiviert, daß das Scutellum auf den Agar aufliegt. Anschließend wird bei 2000 - 5000 Lux (12 h/Tag) bei 75 ± 2°C kultiviert. Embryonen ohne expressionsfähiges PAT-Gen sterben auf diesem Medium innerhalb von 14 Tagen ab, ohne vorher anzukeimen. Tabelle 1 gibt das Verhalten der ersten Hybrid-Filial-Generation (t₁-Hybride) dreier unterschiedlicher Transformanten wieder.

**Tabelle 1**

| Verhalten unreifer Embryonen aus Kreuzungen transgener Vaterpflanzen mit nichttransgenen Inzuchtlinien | | | |
|---|---|---|---|
| Pollenspender | Isolierte Embryonen | absterbende sich entwickelnde Embryonen | |
| Transformante 2 | 20 | 9 | 11 |
| Transformante 5 | 20 | 12 | 8 |
| Transformante 9 | 100 | 47 | 53 |

Die sich entwickelnden Embryonen, die in Gegenwart von 5·10⁻⁴M L-Phosphinothricin auskeimen, entwickeln sowohl normale Wurzeln als auch grüne Blätter auf diesem Medium. Die Pflanzen können analog zu den aus somatischen Embryonen entwickelten in vitro Pflanzen in Erde überführt werden. Diese Pflanzen vererben das PAT-Gen in die t₂-Generation, wenn Pollen der t₁-Pflanzen auf Narben von nichttransgenen Mutterpflanzen gebracht wird.

### b) Sämlingstest

Ausgereifte Samen aus den oben beschriebenen Kreuzungen werden getrocknet (< 15 % Wassergehalt) und ausgesät (20000 Lux 12 h 22°C Tag-/16°C Nachttemperatur). Sobald die Keimpflanzen das 2-3 Blattstadium erreicht haben, werden sie mit einer 1%igen Lösung des Handelsproduktes Basta® in analoger Weise wie die Regeneratpflanzen besprüht (vgl. Beispiel 6).

**Tabelle 2**

| gibt das Ergebnis wieder. | | | |
|---|---|---|---|
| | Sämlinge nach Applikation von 2 kg Glufosinate/ha | | |
| Pollenspender | getestete Sämlinge | ungeschädigt | abgestorben |
| Transformante 9 | 29 | 15 | 14 |
| Transformante 13 | 20 | 11 | 9 |
| Transformante 14 | 39 | 17 | 22 |

Die Spaltungsverhältnisse stehen im Einklang mit der Annahme, daß einige Transformanten nur ein funktionsfähiges PAT-Gen in ihrem Genom besitzen.

Von Transformante Nr. 14 wurden am 12.6.1990 Samen unter den Bedingungen des Budapester Vertrages unter dem Aktenzeichen NCIMB 40291 bei National Collections of Industrial and Marine Bacteria Ltd. (NCIMB), Aberdeen, GB, hinterlegt.

### SEQUENZPROTOKOLL

| | |
|---|---|
| SEQ ID No: | 1 |
| ART DER SEQUENZ: | Nucleotid |
| SEQUENZLÄNGE: | 793 Basenpaare |
| STRANGFORM: | Doppelstrang |
| TOPOLOGIE: | Linear |
| ART DES MOLEKÜLS: | Genom-DNA |

### URSPRÜNGLICHE HERKUNFT

Organismus: Cauliflower Mosaik Virus; E. coli

### UNMITTELBARE EXPERIMENTELLE HERKUNFT

pDH51-Plasmid (Pietrzack et al. Nucleic Acids Research, Band 14, Nummber 14, Seiten 5857-5868 (1986))

| MERKMALE DER SEQUENZ | |
|---|---|
| Lage der Merkmale: | Nucleotid 1-533: Promotorsequenz |
| | Nucleotid 534-609: Polylinker |
| | Nucleotid 610-786: Terminatorsequenz |

### Art der Ermittlung des Merkmals: experimentell

GAATTCCCAT GGAGTCAAAG AATCAAATAG AGGACCTAAC AGAACTCGCC GTAAAGACTG 60
GCGAACAGTT CATACAGAGT CTCTTACGAC TCAATGACAA GAAGAAAATC TTCGTCAACA 120
TGGTGGAGCA CGACACGCTT GTCTACTCCA AAAATATCAA AGATACAGTC TCAGAAGACC 180
AAAGGGCAAT TGAGACTTTT CAACAAAGGG TAATATCCGG AAACCTCCTC GGATTCCATT 240
GCCCAGCTAT CTGTCACTTT ATTGTGAAGA TAGTGGAAAA GGAAGGTGGC TCCTACAAAT 300
GCCATCATTG CGATAAAGGA AAGGCCATCG TTGAAGATGC CTCTGCCGAC AGTGGTCCCA 360
AAGATGGACC CCCACCCACG AGGAGCATCG TGGAAAAAGA AGACGTTCCA ACCACGTCTT 420
CAAAGCAAGT GGATTGATGT GATATCTCCA CTGACGTAAG GGATGACGCA CAATCCCACT 480
ATCCTTCGCA AGACCCTTCC TCTATATAAG GAAGTTCATT TCATTTGGAG AGGACAGGGT 540
ACCCGGGGAT CCTCTAGAGT CGACCTGCAG GCATGCCGCT GAAATCACCA GTCTCTCTCT 600
ACAAATCTAT CTCTCTCTAT AATAATGTGT GAGTAGTTCC CAGATAAGGG AATTAGGGTT 660
CTTATAGGGT TTCGCTCATG TGTTGAGCAT ATAAGAAACC CTTAGTATGT ATTTGTATTT 720
GTAAAATACT TCTATCAATA AAATTTCTAA TTCCTAAAAC CAAAATCCAG TGGGTACCGA 780
GCTCGAATTC AAG 793

| | |
|---|---|
| SEQ ID No: | 2 |
| ART DER SEQUENZ: | Nucleotid mit entsprechendem Protein |
| SEQUENZLÄNGE: | 564 Basenpaare |
| STRANGFORM: | Doppelstrang |
| TOPOLOGIE: | Linear |
| ART DES MOLEKÜLS: | Genom-DNA |

### URSPRÜNGLICHE HERKUNFT

Organismus: Streptomyces viridochromogenes

### UNMITTELBARE EXPERIMENTELLE HERKUNFT: synthetisch

### MERKMALE DER SEQUENZ

Merkmal: Bei dem Protein handelt es sich um eine N-Acetyltransferase Art der Ermittlung des Merkmals: experimentell

GTC GAC ATG TCT CCG GAG AGG AGA CCA GTT GAG ATT AGG CCA GCT ACA 48
Val Asp Met Ser Pro Glu Arg Arg Pro Val Glu Ile Arg Pro Ala Thr
1               5                   10                  15

GCA GCT GAT ATG GCC GCG GTT TGT GAT ATC GTT AAC CAT TAC ATT GAG 96
Ala Ala Asp Met Ala Ala Val Cys Asp Ile Val Asn His Tyr Ile Glu
            20                  25                  30

ACG TCT ACA GTG AAC TTT AGG ACA GAG CCA CAA ACA CCA CAA GAG TGG 144
Thr Ser Thr Val Asn Phe Arg Thr Glu Pro Gln Thr Pro Gln Glu Trp
        35                  40                  45

ATT GAT GAT CTA GAG AGG TTG CAA GAT AGA TAC CCT TGG TTG GTT GCT 192
Ile Asp Asp Leu Glu Arg Leu Gln Asp Arg Tyr Pro Trp Leu Val Ala
    50                  55                  60

GAG GTT GAG GGT GTT GTG GCT GGT ATT GCT TAC GCT GGG CCC TGG AAG 240
Glu Val Glu Gly Val Val Ala Gly Ile Ala Tyr Ala Gly Pro Trp Lys
65                  70                  75                  80

GCT AGG AAC GCT TAC GAT TGG ACA GTT GAG AGT ACT GTT TAC GTG TCA 288
Ala Arg Asn Ala Tyr Asp Trp Thr Val Glu Ser Thr Val Tyr Val Ser
                85                  90                  95

SEQ ID No: 2

CAT AGG CAT CAA AGG TTG GGC CTA GGA TCC ACA TTG TAC ACA CAT TTG 336
His Arg His Gln Arg Leu Gly Leu Gly Ser Thr Leu Tyr Thr His Leu
100 105 110

CTT AAG TCT ATG GAG GCG CAA GGT TTT AAG TCT GTG GTT GCT GTT ATA 384
Leu Lys Ser Met Glu Ala Gln Gly Phe Lys Ser Val Val Ala Val Ile
115 120 125

GGC CTT CCA AAC GAT CCA TCT GTT AGG TTG CAT GAG GCT TTG GGA TAC 432
Gly Leu Pro Asn Asp Pro Ser Val Arg Leu His Glu Ala Leu Gly Tyr
130 135 140

ACA GCC CGG GGT ACA TTG CGC GCA GCT GGA TAC AAG CAT GGT GGA TGG 480
Thr Ala Arg Gly Thr Leu Arg Ala Ala Gly Tyr Lys His Gly Gly Trp
145 150 155 160

CAT GAT GTT GGT TTT TGG CAA AGG GAT TTT GAG TTG CCA GCT CCT CCA 528
His Asp Val Gly Phe Trp Gln Arg Asp Phe Glu Leu Pro Ala Pro Pro
165 170 175

AGG CCA GTT AGG CCA GTT ACC CAG ATC TGA GTC GAC 564
Arg Pro Val Arg Pro Val Thr Gln Ile Val Asp
180 185

## Patentansprüche (Patentansprüche für folgende(n) Vertragsstaat(en): AT, BE, CH, LI, DE, DK, FR, GB, IT, LU, NL, SE)

1. Verfahren zur Herstellung einer transgenen Maiszellinie, dadurch gekenzeichnet, daß
- aus Zellsuspensionen eines auxinautotrophen Maisgenotyps enzymatisch Protoplasten hergestellt werden,
- die Protoplasten mit DNA inkubiert werden und die DNA in die Protoplasten eingeschleust wird, und
- die transgene Maiszellinie aus den DNA enthaltenden Protoplasten selektiert und regeneriert wird.

2. Verfahren nach Anspruch 1, dadurch gekenzeichnet, daß DNA durch Inkubation in Gegenwart von Polyethylenglykol in die Protoplasten eingeschleust wird.

3. Verfahren nach Anspruch 1 oder 2, **dadurch gekennzeichnet, daß** die Protoplasten mit für Phosphinotricinacetyltransferase, Acetyl-CoA-Carboxylase, Glutathion-S-transferase, Hygromycinphosphotransferase, Acetolactatsynthetase, 5-Enolpyruvyl-shikimatphosphatsynthetase, Glutaminsynthetase, Glutamintransaminase, δ-endotoxin von Bacillus thuringiensis, Virus Coat Protein, Chitinase, Phosphoenolpyruvatcarboxylase oder bakterielle Asparaginsynthetase kodierender DNA inkubiert werden.

4. Verfahren nach Anspruch 1 oder 2, **dadurch gekennzeichnet, daß** die Protoplasten mit für Phosphinotricinacetyltransferase kodierender DNA inkubiert werden.

5. Verfahren zur Herstellung transgener Maispflanzen dadurch gekenzeichnet, daß
- aus Zellsuspensionen eines auxinautotrophen Maisgenotyps enzymatisch Protoplasten hergestellt werden,
- die Protoplasten mit DNA inkubiert werden und die DNA in die Protoplasten eingeschleust wird
- die transgene Maiszellinie aus den DNA enthaltenden Protoplasten selektiert und regeneriert wird und
- transgene Maispflanzen aus den transgenen Maiszellinie erhalten werden.

6. Verfahren nach Anspruch 5, dadurch gekenzeichnet, daß DNA durch Inkubation in Gegenwart von Polyethylenglykol in die Protoplasten eingeschleust wird.

7. Verfahren nach Anspruch 5 oder 6, dadurch gekenzeichnet, daß Zellsuspensionen des Genotyps DSM 6009 verwendet werden.

8. Verfahren nach einem oder mehreren der Ansprüche 5 bis 7, weiterhin dadurch gekenzeichnet, daß die transgenen Maispflanzen durch Selbstbestäubung oder durch Fremdbestäubung vermehrt werden.

9. Verfahren nach einem oder mehreren der Ansprüche 5 bis 7, weiterhin dadurch gekenzeichnet, daß die transgene Maispflanzen durch Kreuzung mit Elite-Inzuchtlinien vermehrt werden.

10. Verfahren nach einem oder mehreren der Ansprüche 5 bis 9, dadurch gekenzeichnet, daß die Protoplasten mit für Phosphinotricinacetyltransferase kodierender DNA inkubiert werden.

11. Verfahren zum Schutz von Maispflanzen durch selektive Vernichtung von Unkraut mit Phosphinotricin oder Phosphinotricinalanytalanin auf Anbauflächen, dadurch gekenzeichnet, daß die Anbauflächen mit Pflanzen, erhältlich durch dem Verfahren nach Anspruch 10, bepflanzt werden.

12. Eine transgene Maiszellinie. erhältlich durch ein Verfahren nach Anspruch 1.

13. Die transgene Maiszellinie nach Anspruch 12, hergestellt aus Protoplasten des Genotyps DSM 6009.

14. Die transgene Maiszellinie nach Anspruch 12 oder 13, enthaltend ein Gen kodierend für Phosphinotricinacetyltransferase, Acetyl-CoA-Carboxylase, Glutathion-S-transferase, Hygromycinphosphotransferase, Acetolactatsynthetase, 5-Enolpyruvyl-shikimatphosphatsynthetase, Glutaminsynthetase, Glutamintransaminase, δ-endotoxin von Bacillus thuringiensis, Virus Coat Protein, Chitinase, Phosphoenolpyruvatcarboxylase oder bakterielle Asparaginsynthetase.

15. Die transgene Maiszellinie nach Anspruch 14, enthaltend ein Gen codierend für Phosphinotricinacetyltransferase.

16. Transgene Maispflanzen und Pflanzenteile, regeneriert aus der transgenen Maiszellinie nach Anspruch 12.

17. Nachkommen der transgene Maisplanzen, regeneriert aus der transgenen Maiszellinie nach Anspruch 12.

## Patentansprüche (Patentansprüche für folgende(n) Vertragsstaat(en): ES, GR)

1. Verfahren zur Herstellung einer transgene Maispflanze, dadurch gekenzeichnet daß
- aus Zellsuspensionen eines auxinautotrophen Maisgenotyps enzymatisch Protoplasten hergestellt werden,
- die Protoplasten mit DNA inkubiert werden und die DNA in die Protoplasten eingeschleust wird,
- die transgene Maiszellinie aus den DNA enthaltenden Protoplasten selektiert und regeneriert wird, und
- aus der Maiszellinie transgene Pflanzenteile bzw. Pflanzen regeneriert werden.

2. Verfahren nach Anspruch 1, dadurch gekenzeichnet, daß DNA durch Inkubation in Gegenwart von Polyethylenglykol in die Protoplasten eingeschleust wird.

3. Verfahren nach Anspruch 1 oder 2, **dadurch gekennzeichnet, daß** Protoplasten des Genotyps DSM 6009 verwendet werden.

4. Verfahren nach einem oder mehreren der Ansprüche 1 bis 3, weiterhin dadurch gekenzeichnet, daß die transgenen Planzen durch Selbstbestäubung oder durch Fremdbestäubung vermehrt werden.

5. Verfahren nach einem oder mehreren der Ansprüche 1 bis 4, weiterhin dadurch gekenzeichnet, daß die transgenen Maispflanzen durch Kreuzung mit Elite-Inzuchtlinien vermehrt werden.

6. Verfahren nach einem oder mehreren der Ansprüche 1 bis 5, dadurch gekenzeichnet,, daß die Protoplasten
- mit für Phosphinotricinacetyltransferase kodierender DNA inkubiert werden,
- die transgene Maiszellinie aus den DNA enthaltenden Protoplasten selektiert und regeneriert wird und
- aus der Maiszellinie trangene Pflanzenteile bzw. Pflanzen regeneriert werden.

7. Verfahren zum Schutz von Maispflanzen durch selektieve Vernichtung von Unkraut mit Phosphinotricin oder Phosphinotricinalanylalanin auf Anbauflächen, dadurch gekenzeichnet, daß die Anbauflächen mit Pflanzen erhältlich aus dem Verfahren nach Anspruch 6 bepflanzt werden.

## Claims (Claims for the following Contracting State(s): AT, BE, CH, LI, DE, DK, FR, GB, IT, LU, NL, SE)

1. Process for the production of a transgenic corn cell line, **characterized in that**
- protoplasts are produced enzymatically from cell suspensions of an auxin autotrophic genotype,
- the protoplasts are incubated with DNA and the DNA is introduced into the protoplasts, and
- the trangenic corn cell line is selected and regenerated from the protoplasts comprising the DNA.

2. Process according to claim 1, **characterized in that** DNA is introduced into the protoplasts by incubation in the presence of polyethylene glycol.

3. Process according to claim 1 or 2, **characterized in that** the protoplasts are incubated with DNA encoding phosphinotricin acetyltransferase, acetyl-CoA-carboxylase, glutathion-S-transferase, hygromycin phosphotransferase, acetolactate synthase, 5-enolpyruvate-shikimate phosphosynthetase, glutamine synthetase, glutamine transaminase, δ-endotoxin from Bacillus thuringiensis, virus coat protein, chitinase, phosphoenolpuryvate carboxylase or bacterial asparagine synthetase.

4. Process according to claim or 2, **characterized in that** the protoplasts are incubated with DNA encoding phosphinotricin acetyltransferase.

5. Process for the production of transgenic corn plants, **characterized in that**
- protoplasts are produced enzymatically from cell suspensions of an auxin autotrophic genotype,
- the protoplasts are incubated with DNA and the DNA is introduced into the protoplasts,
- the trangenic corn cell line is selected and regenerated from the protoplasts comprising the DNA, and
- transgenic corn plants are obtained from the transgenic corn cell line.

6. Process according to claim 5, **characterized in that** DNA is introduced into the protoplasts by incubation in the presence of polyethylene glycol.

7. Process according to claim 5 or 6, **characterized in that** cell suspensions from the genotype DSM 6009 are used.

8. Process according to any one of claims 5 to 7, further **characterized in that** the transgenic com plants are propagated by self pollination or by cross pollination.

9. Process according to any one of claims 5 to 7, further **characterized in that** the transgenic corn plants are propagated by crossing with elite inbred lines.

10. Process according to any one of claims 5 to 9, **characterized in that** the protoplasts are incubated with DNA encoding phosphinotricin acetyltransferase.

11. Process for the protection of maize plants by selective destruction of weeds with phosphinotricin or phosphinotricinalanylalanine on cultivable land, **characterized in that** the cultivable land is planted with plants that are obtainable by the process of claim 10.

12. A transgenic corn cell line obtainable by the process of claim 1.

13. The trangenic corn cell line according to claim 12, produced from protoplasts of the genotype DSM 6009.

14. The transgenic corn cell line according to claim 12 or 13, comprising a gene encoding phosphinotricin acetyltransferase, acetyl-CoA-carboxylase, glutathion-S-transferase, hygromycin phosphotransferase, acetolactate synthase, 5-enolpyruvate-shikimate phosphosynthetase, glutamine synthetase, glutamine transaminase, δ-endotoxin from Bacillus thuringiensis, virus coat protein, chitinase, phosphoenolpuryvate carboxylase or bacterial asparagine synthetase.

15. The transgenic corn cell line according to claim 14, comprising a gene encoding phosphinotricin acetyltransferase.

16. Transgenic corn plants and parts of plants, regenerated from the transgenic corn cell line according to claim 12.

17. Progeny of transgenic corn plants, regenerated from the transgenic corn cell line according to claim 12.

## Claims (Claims for the following Contracting State(s): ES, GR)

1. Process for the production of a transgenic com plants, **characterized in that**
- protoplasts are enzymatically produced from cell suspensions of an auxin autotrophic genotype,
- the protoplasts are incubated with DNA and the DNA is introduced into the protoplasts,
- the trangenic corn cell line is selected and regenerated from the protoplasts comprising the DNA, and
- transgenic plant parts, respectively plants are regenerated from the corn cell line.

2. Process according to claim 1, **characterized in that** DNA is introduced into the protoplasts by incubation in the presence of polyethylene glycol.

3. Process according to claim 1 or 2, **characterized in that** protoplasts from the genotype DSM6009 are used.

4. Process according to any one of claims 1 to 3, further **characterized in that** the transgenic corn plants are propagated by self pollination or by cross pollination.

5. Process according to any one of claims 1 to 4, further **characterized in that** the transgenic com plants are propagated by crossing with elite inbred lines.

6. Process according to any one of claims 1 to 5, **characterized in that** the protoplasts
- are incubated with DNA encoding phosphinotricin acetyltransferase,
- the transgenic com cell line is selected and regenerated from the protoplasts comprising the DNA,and
- transgenic plant parts, respectively plants are regenerated from the com cell line.

7. Process for the protection of maize plants by selective destruction of weeds with phosphinotricin or phosphinotricinalanylalanine on cultivable land, **characterized in that** the cultivable land is planted with plants that are obtainable by the process of claim 6.

## Revendications (Revendications pour l'(les) Etat(s) contractant(s) suivant(s): AT, BE, CH, LI, DE, DK, FR, GB, IT, LU, NL, SE)

1. Procédé pour produire une culture cellulaire de maïs transgénique, **caracterisé en ce que**
- des protoplastes sont produits à partir d'une suspension des cellules d'un génotype de maïs autotrophe en auxine, par voie enzymatique,
- les protoplastes sont incubés en présence d'ADN et l'ADN est introduit dans les protoplastes, et
- une culture cellulaire de maïs transgénique est sélectionnée et régénérée à partir des protoplastes contenant l'ADN.

2. Procédé selon la revendication 1, **caractérisé en ce que** l'ADN est introduit dans les protoplastes par incubation en présence de polyéthylène glycol.

3. Procédé selon la revendication 1 ou 2, **caractérisé en ce que** les protoplastes sont incubés en présence d'ADN codant pour phosphinotricine acétyltransférase, acétyl-CoA-carboxylase, glutathione-S-transférase, hygromycine phosphotransférase, acétolactate synthase, 5-enolpyruvate-shikimate phosphosynthétase, glutamine synthétase, glutamine transaminase, δ-endotoxine de Bacillus thuringiensis, protéine enveloppe de virus, chitinase, phospho enolpuryvate carboxylase ou asparagine synthétase bactérienne.

4. Procédé selon la revendication 1 ou 2, **caractérisé en ce que** les protoplastes sont incubés en présence d'ADN codant pour phosphinotricine acétyltra nsfé rase.

5. Procédé pour produire des plantes de maïs transgéniques, **caractérisé en ce que**
- des protoplastes sont produits à partir d'une suspension des cellules d'un génotype de maïs autotrophe en auxine, par voie enzymatique,
- les protoplastes sont incubés en présence d'ADN et l'ADN est introduit dans les protoplastes,
- une culture cellulaire de maïs transgénique est selectionnée et régénérée à partir des protoplastes contenant l'ADN, et
- des plantes de maïs transgéniques sont obtenues à partir de la culture cellulaire de maïs transgénique.

6. Procédé selon la revendication 5, **caractérisé en ce que** l'ADN est introduit dans les protoplastes par incubation en présence de polyéthylène glycol.

7. Procédé selon la revendication 5 ou 6, **caractérisé en ce que** les suspensions des cellules du génotype DSM6009 sont utilisés.

8. Procédé selon l'une quelconque des revendications 5 à 7, **caracterisé en ce que** les plantes de maïs transgéniques sont multipliées par autofécondation ou par pollinisation croissée.

9. Procédé selon l'une quelconque des revendications 5 à 7, **caracterisé en ce que** les plantes de maïs transgéniques sont multipliées par croissement avec des lignées élites inbred.

10. Procédé selon l'une quelconque des revendications 5 à 9, **caracterisé en ce que** les protoplastes sont incubés en présence d'ADN codant pour phosphinotricine acétyltransferase.

11. Procédé pour la protection des plantes de maïs par destruction sélective des mauvaises herbes avec phosphinotricine ou phosphinotricinalanylalanine sur la terre cultivée, **caractérisé en ce que** la terre cultivée est planté avec des plantes qui peuvent être obtenues par le procédé selon la revendication 10.

12. Une culture cellulaire de maïs transgénique, qui peut être obtenue par le procédé selon la revendication 1.

13. La culture cellulaire de maïs transgénique selon la revendication 12, produite à partir des protoplastes du génotype DSM 6009.

14. La culture cellulaire de maïs transgénique selon la revendication 12 ou 13, comprenant un gène codant pour phosphinotricine acétyltransferase, acétyl-CoA-carboxylase, glutathione-S-transférase, hygromycine phosphotransférase, acétolactate synthase, 5-enolpyruvate-shikimate phosphosynthétase, glutamine synthétase, glutamine transaminase, δ-endotoxine de Bacillus thuringiensis, protéine enveloppe de virus, chitinase, phospho enolpuryvate carboxylase ou asparagine synthétase bactérienne.

15. La culture cellulaire de maïs transgénique selon la revendication 14, comprenant un gène codant pour phosphinotricine acétyltransferase.

16. Des plantes de maïs transgéniques et des parties de ces plantes, régénérées à partir de la culture cellulaire de maïs transgénique selon la revendication 12.

17. Progéniture des plantes de maïs, régénérée a partir de la culture cellulaire de maïs transgénique selon la revendication 12.

## Revendications (Revendications pour l'(les) Etat(s) contractant(s) suivant(s): ES, GR)

1. Procédé pour produire une plante de maïs transgénique, **caractérisé en ce que**
- des protoplastes sont produits à partir d'une suspension des cellules d'un génotype de maïs autotrophe en auxine, par voie enzymatique,
- les protoplastes sont incubés en présence d'ADN et l'ADN est introduit dans les protoplastes,
- une culture cellulaire de maïs transgénique est selectionnée et régénérée à partir des protoplastes contenant l'ADN, et
- des parties de plantes transgéniques ou des plantes sont obtenues à partir de la culture cellulaire de maïs transgénique.

2. Procédé selon la revendication 1, **caractérisé en ce que** l'ADN est introduit dans les protoplastes par incubation en présence de polyéthylène glycol.

3. Procédé selon la revendication 1 ou 2, **caractérisé en ce que** des protoplastes du génotype DSM6009 sont utilisés.

4. Procédé selon l'une quelconque des revendications 1 à 3, **caracterisé en ce que** les plantes de maïs transgéniques sont multipliées par autofécondation ou par pollinisation croissée.

5. Procédé selon l'une quelconque des revendications 1 à 4, **caracterisé en ce que** les plantes de maïs transgéniques sont multipliées par croissement avec des lignées élites inbred.

6. Procédé selon l'une quelconque des revendications 1 à 5, **caracterisé en ce que**
- les protoplastes sont incubés en présence d'ADN codant pour phosphinotricine acétyltransférase,
- une culture cellulaire de maïs transgénique est selectionnée et régénérée à partir des protoplastes contenant l'ADN, et
- des parties de plantes transgéniques ou des plantes sont obtenues à partir de la culture cellulaire de maïs transgénique.

7. Procédé pour la protection de maïs par la destruction sélective des mauvaises herbes avec phosphinotricine ou phosphinotricinalanylalanine sur la terre cultivée, **caracterisé en ce que** la terre cultivée est planté avec des plantes qui peuvent être obtenues par le procédé selon la revendication 6.
